# EUROPÄISCHE PATENTSCHRIFT

(11) **EP 1 054 866 B1**
(45) Veröffentlichungstag und Bekanntmachung des Hinweises auf die Patenterteilung: **16.04.2003**
(21) Anmeldenummer: 99906194.8
(22) Anmeldetag: 22.01.1999
(51) Int. Cl.: C07D 213/20, C07B 45/02

(54) **SYNTHESE VON 1-(2-SULFOETHYL)-PYRIDINIUMBETAIN**
SYNTHESIS OF 1-(2-SULPHOETHYL)-PYRIDINIUM BETAINE
SYNTHESE DE BETAINE DE 1-(2-SULFOETHYL)-PYRIDINIUM

(30) Priorität: 11.02.1998 DE 19805487
(43) Veröffentlichungstag der Anmeldung: 29.11.2000
(73) Patentinhaber: BASF AKTIENGESELLSCHAFT, 67056 Ludwigshafen (DE)
(72) Erfinder: KAPPES, Elisabeth, D-67117 Limburgerhof (DE); BRODT, Gregor, D-64646 Heppenheim (DE); KRÖNER, Rudi, D-68167 Mannheim (DE); WAGNER, Norbert, D-67112 Mutterstadt (DE); BOGENSTÄTTER, Thomas, D-67098 Bad Dürkheim (DE); HILDEBRANDT, Sören, D-67346 Speyer (DE)
(74) Vertreter: Isenbruck, Günter, Dr.
(86) Internationale Anmeldenummer: EP9900431
(87) Internationale Veröffentlichungsnummer: WO99041236

(56) Entgegenhaltungen:
- WO-A-91/16474
- FR-A- 2 270 241
- US-A- 3 131 189
- US-A- 3 275 672
- LE BERRE, ANDRE ET AL: "Addition of tertiary amine salts to electrophilic ethylenic compounds. II. Betaines and quaternary salts from.alpha.,.beta.-unsaturated acids" BULL. SOC. CHIM. FR. (1973), (7-8)(PT. 2), 2404-8 CODEN: BSCFAS, XP002103472 in der Anmeldung erwähnt

## Beschreibung

Die Erfindung betrifft die Herstellung von 1-(2-Sulfoethyl)-pyridiniumbetain.

1-(2-Sulfoethyl)-pyridiniumbetain (PES) gehört zur Verbindungsklasse der Sulfobetaine. Dabei handelt es sich um innere Salze (= zwitterionische Verbindungen), in denen sich die positive Ladung am Stickstoff-Atom und die negative Ladung an der Sulfonat-Gruppe befindet. PES spielt z.B. eine wichtige Rolle als Spitzenglanzbildner bei der elektrolytischen Nickel-Abscheidung.

In der WO 91/16474 wird der Einsatz von PES als Spitzenglanzbildner bei der Abscheidung von Nickel aus sauren Nickelbädern beschrieben. Spitzenglanzbildner dienen dazu, rauhe Oberflächen, wie sie bei der Abscheidung von Nickel aus Nickel-Elektrolyten erhalten werden, einzuebnen, ohne den Niederschlag zu verspröden. Sie werden zur Erhöhung der Qualität von Nickel-Überzügen praktisch immer zusammen mit einem oder mehreren Grundglanzbildnern angewendet, die eine glänzende statt einer matten Nickelabscheidung bewirken.

Die Herstellung von PES wurde bereits in der Literatur beschrieben.

In der US 3,131,189 wird die Synthese von quartären Ammoniumbetainsalzen aus Carbylsulfat oder Derivaten des Carbylsulfats und tertiären Aminen offenbart. Unter anderem wird die Synthese von PES aus Pyridin und Carbylsulfat bei Raumtemperatur in Dichlorethan beschrieben.

Auch in J. Org. Chem. 29, 2489 (1964) wird die Reaktion von Carbylsulfat mit Pyridin in Dichlorethan beschrieben. Dabei wird PES in 60%iger Ausbeute erhalten.

In den Verfahren gemäß der beiden vorstehend genannten Veröffentlichungen werden jedoch nur kleine Ansatzgrößen im Bereich von ca. 5g PES realisiert. Nachteilig ist ferner die Verwendung von organischen Lösungsmitteln wie Dichlorethan, von dem berichtet wird, daß es im Tierversuch krebserzeugend sei.

Eine weitere, zweistufige Synthese von PES wird in J. Org. Chem. 26, 4520 (1961) behandelt. Im ersten Schritt erfolgt die Umsetzung von Pyridin mit 1,2-Dibromethan, wobei ein bromethylsubstituiertes Pyirdiniumbromid entsteht, das unter Zugabe von Natriumsulfit zu PES umgesetzt wird.

Die FR-B 2 270 241 beschreibt ein Verfahren zur Sulfoethylierung tertiärer Amine unter Verwendung von aliphatischen Estern der Ethensulfonsäure als Sulfonierungsmittel. Die Produkte sind die Sulfoethylbetaine der tertiären Amine. Dabei werden Ausbeuten von bis zu 95% für PES erzielt. Nachteilig ist, daß die im Vergleich zu Vinylsulfonsäure teureren Vinylsulfonsäureester eingesetzt werden. Diese müssen erst durch die Veresterung der entsprechenden Säure mit Alkylorthoformiaten, Chloroformiaten oder Alkylchlorosulfiten oder, wie in H. Distler, Angew. Chem. 77, 291 (1965) beschrieben, durch Umsetzung von Alkoholaten mit β-Chlorethansulfonsäurechlorid hergestellt werden. Ein weiterer Nachteil ist der als Abfall in stöchiometrischen Mengen anfallende Esteralkohol.

In Bull. Soc. Chim. Fr. 1973, 7-8, 2404 wird die Bildung von Sulfobetainen, auch PES, aus Ethensulfonsäure bzw. ihrem Salz und tertiären Aminen beschrieben. Die Synthese von PES, ausgehend von Ethensulfonsäure, wird als schwierig bezeichnet. Durch Erhitzen des lösungsmittelfreien Salzes Pyridiniumvinylsulfonat auf maximal 150°C kann PES in Spuren erhalten werden. Eine leicht erfolgreichere Umsetzung, die jedoch sehr langsam ist, wird durch Umsetzung von Pyridin mit Vinylsulfonat in siedender Essigsäure erzielt.

US-A-3 275 672 beschreibt ein verfahren zur Herstellung von Sulfonsäureestern, insbesondere von vinylsulfonsäureestern. Die Herstellung erfolgt durch umsetzung von Carbylsulfat mit einem Alkohol.

Aufgabe der Erfindung ist es, eine kostengünstige, großtechnisch relevante Synthese für PES bereitzustellen. Eine weitere Aufgabe, die sich aus oben genannten Nachteilen ergibt, ist die Vermeidung des Einsatzes chlorierter organischer Lösungsmittel, die eventuell krebserzeugend sind.

Erfindungsgmäß wird diese Aufgabe durch ein Verfahren zur Herstellung von PES gelöst, in dem Pyridin mit einem Sulfoethylierungsmittel aus der Gruppe Carbylsulfat, Ethionsäure bzw. ihrem Salz und Vinylsulfonsäure bzw. Vinylsulfonat umgesetzt wird, wobei die Umsetzung in wäßriger Lösung mit einem Gehalt von mindestens 20 Gew.-% Wasser erfolgt.

Durch das erfindungsgemäße Verfahren wird eine kostengünstige Synthese bereitgestellt, die in großtechnischem Maßstab durchführbar ist. Es kann dabei auf teure, eventuell krebserzeugende chlorierte organische Lösungsmittel verzichtet werden und die Synthese kann als "Eintopfreaktion", d.h. in einem Schritt, ohne Isolierung von Zwischenprodukten, durchgeführt werden.

Die Herstellung von PES kann in einer Ausführungsform der Erfindung durch die Umsetzung von Pyridin mit einem Sulfoethylierungsmittel aus der Gruppe Carbylsulfat, Ethionsäure bzw. ihrem Salz und Vinylsulfonsäure bzw. Vinylsulfonat in wäßriger Lösung erfolgen.

Unter einer wäßrigen Lösung ist dabei eine Reaktionsmischung mit einem Gehalt von mindestens 20 Gew.-%, bevorzugt zwischen 20 Gew.-% und 80 Gew.-%, besonders bevorzugt zwischen 30 Gew.-% und 70 Gew.-%, ganz besonders bevorzugt 45 Gew.-% und 65 Gew.-%, Wasser zu verstehen.

Bei Durchführung der Umsetzung in wäßriger Lösung wird im allgemeinen ein pH-Wert im Bereich von 1,0 bis 11,0, bevorzugt 4,0 bis 9,0, besonders bevorzugt 5,0 bis 8,0, eingehalten, der mit Säure, bevorzugt Schwefelsäure, oder Alkalilauge eingestellt wird. Dabei sind Schwankungen des pH-Wertes von ca. ± 2 innerhalb der angegebenen Ober- und Untergrenzen möglich.

Das Sulfoethylierungsmittel wird mit Pyridin in einem Molverhältnis von 1:0,1 bis 1:3, bevorzugt von 1:0,5 bis 1:2 , besonders bevorzugt von 1:0,6 bis 1:1, gemischt. Die Mischung wird, im allgemeinen unter Rühren, auf 20 bis 250°C, bevorzugt auf 80 bis 160°C, besonders bevorzugt auf 130 bis 160°C, erhitzt. Der Druck beträgt im allgemeinen 1.013 bar (1 atm) bis 202.65 bar (200 atm.) bevorzugt 1.013 bar (1 am), bis 50.6625 bar (50 atm.), besonders bevorzugt 2.0265 bar (2 atm.) bis 10.1325 bar (10 atm). Ganz besonders bevorzugt wird die Umsetzung bei einem Eigendruck von ca. 3.0397 bar (3 atm.) bis 8.106 bar (8 atm) in einem Autoklaven durchgeführt.

Die Reaktionszeit liegt, je nach den gewählten Bedingungen, zwischen 3 und 40 Stunden, bevorzugt zwischen 4 und 20 Stunden.

Als Reaktionsprodukt erhält man eine wasserklare bis bräunliche wäßrige Lösung, die PES und Alkalisulfat enthält. Wird zur Einstellung des pH-Wertes eine von Schwefelsäure verschiedene Säure eingesetzt, so enthält die Lösung anstelle von Alkalisulfat das der Säure entsprechende Salz. Daneben können nicht umgesetztes Sulfoethylierungsmittel und Pyridin enthalten sein. In Abhängigkeit von den gewählten Ausgangsstoffen und Reaktionsbedingungen können weitere Nebenprodukte wie etwaige Nebenprodukte aus der Synthese des Sulfoethylierungsmittels sowie gegebenenfalls während der Reaktion entstandene Hydrolyseprodukte enthalten sein.

Das je nach Reaktionsbedingungen anfallende Salz kann aus den wäßrigen Produktlösungen durch allgemein bekannte Verfahrensschritte zumindest teilweise entfernt werden. So kann der Gehalt an Alkalisulfat in den wäßrigen Produktlösungen z.B. durch Abkühlen und anschließende Filtration reduziert werden. Das bei einer eventuellen unvollständigen Umsetzung verbleibende Vinylsulfonat kann in der Regel für den Einsatz bei einer späteren Verwendung von PES als Spitzenglanzbildner in der Lösung verbleiben, nicht umgesetztes Pyridin kann durch Wasserdampfdestillation entfernt und gegebenenfalls in nachfolgenden Ansätzen wiedereingesetzt werden. Die PES enthaltende Lösung kann nach i.a. bekannten Methoden, z.B. Sprühtrocknung. in eine feste Form überführt werden. Ebenso kann PES aus diesen Lösungen mit Hilfe von dem Fachmann bekannten Methoden isoliert werden.

Bei Einsatz von Carbylsulfat als Sulfoethylierungsmittel in wäßriger Lösung wird, zusätzlich zu den vorstehend beschriebenen Verfahrensschritten, Carbylsulfat zunächst mit 10-50 Gew.-%iger Alkalilauge bei 10 bis 70°C in einer aus EP-B-0 054 142 bekannten Reaktion umgesetzt. Während der Carbylsulfatzugabe sind bevorzugt ein pH-Wert größer oder gleich 7 und eine Temperatur kleiner oder gleich 50°C einzuhalten.

Nach beendeter Carbylsulfat-Zugabe wird die Reaktionsmischung wie oben beschrieben erhitzt, wobei der pH-Wert mit wäßriger Alkalilauge zunächst bei größer 7,0, bevorzugt größer 9.0 gehalten wird, und anschließend durch Zugabe von Säure ein pH-Wert von 1,0 bis 9,0, bevorzugt von 4,0 bis 8,5, besonders bevorzugt von 5,0 bis 8,0 eingestellt. Dabei sind Schwankungen des pH-Wertes von ca. ± 2 innerhalb der angegebenen Ober- und Untergrenzen möglich.

Die weitere Synthese erfolgt unter den oben beschriebenen Reaktionsbedingungen. Hierbei ist es gleichgültig, ob das Pyridin bereits zu Beginn der Umsetzung zugegeben wird, oder nach der Umsetzung von Carbylsulfat und Alkalilauge.

Bei dieser Umsetzung können die folgenden Reaktionswege einzeln oder nebeneinander ablaufen:
a) die direkte Bildung von PES aus Carbylsulfat.
b) die Bildung von Ethionsäure bzw. ihrem Salz aus Carbylsulfat, welche in situ direkt zu PES reagiert oder über aus der Ethionsäure bzw. ihrem Salz entstehendem Vinylsulfonat,
c) die Bildung von Vinylsulfonat aus Carbylsulfat, welches in situ zu PES umgesetzt wird,

Die bei der Umsetzung von Carbylsulfat und Pyridin unter den erfindungsgemäßen Reaktionsbedingungen als Zwischenprodukte entstehenden Verbindungen wie Vinylsulfonsäure bzw. Vinylsulfonat und Ethionsäure bzw. ihr Salz können auch direkt als Sulfoethylierungsmittel in der Synthese von PES unter den oben beschriebenen Reaktionsbedingungen eingesetzt werden. Dabei können Vinylsulfonsäure bzw. Vinylsulfonat und Ethionsäure bzw. ihr Salz nicht nur aus Carbylsulfat, sondern auch aus anderen Ausgangsstoffen erhalten werden. Vinylsulfonat kann beispielsweise durch Umsetzung von Ethanol und Schwefeltrioxid, die aus J. Am. Chem. Soc. 76, 5361 (1954) und US 3,637,793 bekannt ist, erhalten werden. Bei der Umsetzung tritt Ethionsäure als Zwischenprodukt auf.

Besonders bevorzugt ist der Einsatz von Vinylsulfonat als Sulfoethylierungsmittel. Bevorzugt wird das technisch erhältliche Vinylsulfonsäure-Natriumsalz, besonders bevorzugt als 10-40 Gew.-%ige, ganz besonders bevorzugt als 25-30 Gew.-%ige wäßrige Lösung, eingesetzt.

Dabei ist unter einer technisch erhältlichen Vinylsulfonsäure-Natriumsalz-Lösung eine die üblichen Nebenprodukte enthaltende Lösung zu verstehen.

Der pH-Wert wird durch Zugabe von Säure, bevorzugt einem Äquivalent, auf einen pH-Wert im Bereich von 1,0 bis 9,0, bevorzugt 4,0 bis 9,0, besonders bevorzugt 5,0 bis 8,0 eingestellt und während der Umsetzung durch kontinuierliche oder portionsweise Zugabe von Säure eingehalten. Desweiteren kann die benötigte Säuremenge in einer Portion zugesetzt werden. Dabei sind Schwankungen des pH-Wertes von ca. ± 2 innerhalb der angegebenen Ober- und Untergrenzen möglich. Besonders bevorzugt ist eine pH-geregelte Dosierung der Säure.

Als Säure wird bevorzugt Schwefelsäure verwendet. Alternativ ist es möglich. eine schwache Säure, bevorzugt wäßrige Essigsäure, als Lösungsmittel zu verwenden. Eine zusätzliche Dosierung der Säure, bzw. pH-Regelung entfällt damit. Die weitere Umsetzung erfolgt unter den oben beschriebenen Reaktionsbedingungen.

Eine weitere Alternative ist der Einsatz von wasserfreier Essigsäure als Lösungsmittel bei Durchführung der Umsetzung unter Eigendruck bei einer Temperatur oberhalb des Siedepunkts der Essigsäure, bevorzugt bei 130°C bis 160°C. Eine zusätzliche Dosierung der Säure, bzw. pH-Regelung entfällt.

Es können Polymerisationsinhibitoren zugesetzt werden, um eine Polymerisation des Vinylsulfonats zu verhindern.

Die nachfolgenden Beispiele erläutern die Erfindung zusätzlich.

### Beispiel 1

520g 25 Gew.-%ige wäßrige technische Vinylsulfonat-Lösung und 79g Pyridin werden mit 96 Gew.-%iger Schwefelsäure auf pH 5,9 gestellt und unter Rühren im Eigendruck auf 150°C erhitzt. Es werden kontinuierlich ca. 39g einer 96 Gew.-%igen Schwefelsäure zugegeben, so daß der pH-Wert zwischen 7,5 bis 5,0 (gemessen bei Raumtemperatur) liegt. Nach 10 Stunden erhält man eine wäßrige Lösung, die 150g PES, 26g nicht umgesetztes Vinylsulfonat und 56,8g Natriumsulfat enthält. Daneben sind in geringem Maße Nebenprodukte aus der technischen Synthese von Vinylsulfonat vorhanden. Nicht umgesetztes Pyridin wird durch Wasserdampfdestillation entfernt und steht für nachfolgende Umsetzungen zur Verfügung.

### Beispiel 2

79g Pyridin, 200g 50 Gew.-%ige wäßrige NaOH-Lösung und 188g Carbylsulfat (in Stücken oder geschmolzen) werden in 210g Wasser gegeben (exotherme Reaktion!) und gegebenenfalls mit NaOH auf pH größer 9 eingestellt. Während der Carbylsulfatzugabe sollte der pH-Wert nicht unter pH 7,0 und die Temperatur nicht über 50°C steigen. Man erhitzt in einem Autoklaven auf 145°C bis 150°C unter Eigendruck und stellt anschließend durch Zugabe von 96 Gew.-%iger Schwefelsäure auf pH 5,0 bis 7,5 ein. Nach ca. 10 Stunden erhält man eine Lösung, die 140g PES, 17,5g Ethionsäure-di-Natriumsalz, 23,4g Vinylsulfonat und 170g Natriumsulfat enthält.

### Beispiel 3

520g 25 Gew.-%ige wäßrige technische Vinylsulfonat-Lösung und 63,2g Pyridin werden unter Rühren in Eigendruck auf 145°C erhitzt. Es wird mit 96 Gew.-%iger Schwefelsäure auf pH 7,5 bis 5,0 gestellt. Nach ca. 7 Stunden erhält man eine wäßrige Lösung, die als Hauptkomponenten 142g PES, 31,2g Vinylsulfonat und ca. 54g Natriumsulfat enthält. Enthält die eingesetzte technische Vinylsulfonat-Lösung bereits Natriumsulfat, so erhöht sich der gefundene Sulfat-Wert entsprechend. Nicht umgesetztes Pyridin wird durch Wasserdampfdestillation entfernt und steht für nachfolgende Umsetzungen zur Verfügung. Die resultierende Lösung wird auf unter 10°C abgekühlt und ausgefallenes Natriumsulfat wird abfiltriert.

## Patentansprüche

1. Verfahren zur Herstellung von 1-(2-Sulfoethyl)-pyridiniumbetain (PES) in welchem Pyridin mit einem Sulfoethylierungsmittel aus der Gruppe Carbylsulfat, Ethionsäure bzw. ihrem Salz und Vinylsulfonsäure bzw. Vinylsulfonat umgesetzt wird, wobei die Umsetzung in wäßriger Lösung mit einem Gehalt von mindestens 20 Gew.-% Wasser erfolgt.

2. Verfahren nach Anspruch 1, **dadurch gekennzeichnet, daß** Pyridin und das Sulfoethylierungsmittel in einem Molverhältnis von 0,1:1 bis 3:1 umgesetzt werden.

3. Verfahren nach einem der Ansprüche 1 oder 2, **dadurch gekennzeichnet, daß** der pH-Wert auf 1,0 bis 11,0 eingestellt und während der Umsetzung in diesem Bereich eingehalten wird.

4. Verfahren nach einem der Anprüche 1 bis 3, **dadurch gekennzeichnet, daß** die Umsetzung bei einer Temperatur von 20°C bis 250°C und einem Druck von 1.013 bar (1 atm.) bis 202.65 bar (200 atm.) erfolgt.

5. Verfahren nach einem der Ansprüche 1 bis 4, **dadurch gekennzeichnet, daß** als Sulfoethylierungsmittel Carbylsulfat eingesetzt wird.

6. Verfahren nach einem der Ansprüche 1 bis 4, **dadurch gekennzeichnet, daß** als Sulfoethylierungsmittel Ethionsäure bzw. ihr Salz eingesetzt wird.

7. Verfahren nach einem der Anprüche 1 bis 4, **dadurch gekennzeichnet, daß** als Sulfoethylierungsmittel Vinylsulfonsäure bzw. Vinylsulfonat eingesetzt wird.

8. Verfahren nach Anspruch 7, **dadurch gekennzeichnet, daß** Vinylsulfonat in Form einer technisch erhältlichen 10 bis 40 Gew.-%igen Vinylsulfonsäure-Natriumsalzlösung eingesetzt wird.

9. Verfahren nach Anspruch 8, **dadurch gekennzeichnet, daß** der pH-Wert durch Zugabe von Säure eingestellt und eingehalten wird.

10. Verfahren nach Anspruch 9, **dadurch gekennzeichnet, daß** als Säure Schwefelsäure eingesetzt wird, welche in einer Portion zugegeben wird.

## Claims

1. A process for preparing 1-(2-sulfoethyl)pyridinium betaine (PES) wherein pyridine is reacted with a sulfoethylating agent from the group consisting of carbyl sulfate, ethionic acid and its salt and vinylsulfonic acid and vinylsulfonate, the reaction taking place in aqueous solution having a water content of at least 20% by weight.

2. A process as claimed in claim 1, wherein pyridine and the sulfoethylating agent are reacted in a molar ratio of from 0.1:1 to 3:1.

3. A process as claimed in either of claims 1 and 2, wherein the pH is adjusted to from 1.0 to 11.0 and is maintained within this range during the reaction.

4. A process as claimed in any of claims 1 to 3, wherein the reaction takes place at a temperature of from 20°C to 250°C under a pressure of from 1.013 bar (1 atm) to 202.65 bar (200 atm).

5. A process as claimed in any of claims 1 to 4, wherein carbyl sulfate is used as sulfoethylating agent.

6. A process as claimed in any of claims 1 to 4, wherein ethionic acid and/or its salt is used as sulfoethylating agent.

7. A process as claimed in any of claims 1 to 4, wherein vinylsulfonic acid and/or vinylsulfonate is used as sulfoethylating agent.

8. A process as claimed in claim 7, wherein vinylsulfonate is used in the form of an industrially obtainable solution of the sodium salt of vinylsulfonic acid with a concentration of from 10 to 40% by weight.

9. A process as claimed in claim 8, wherein the pH is established and maintained by adding acid.

10. A process as claimed in claim 9, wherein the acid used is sulfuric acid which is added in one portion.

## Revendications

1. Procédé de préparation de la bétaïne du 1-(2-sulfoéthyl)-pyridinium (PES) dans lequel on fait réagir de la pyridine avec un agent de sulfoéthylation du groupe formé par le carbylsulfate, l'acide éthionique ou son sel et l'acide vinylsulfonique ou le vinylsulfonate, la réaction se produisant dans une solution aqueuse contenant une teneur en eau d'au moins 20% en poids.

2. Procédé selon la revendication 1, **caractérisé en ce que** l'on fait réagir la pyridine et l'agent de sulfoéthylation dans un rapport molaire de 0,1:1 à 3:1.

3. Procédé selon l'une quelconque des revendications 1 ou 2, **caractérisé en ce que** l'on ajuste le pH entre 1,0 et 11,0 et qu'on le maintient dans cette gamme pendant la réaction.

4. Procédé selon l'une quelconque des revendications 1 à 3, **caractérisé en ce que** la réaction se produit à une température de 20°C à 250°C et sous une pression de 1,013 bar (1 atm.) à 202,65 bars (200 atm.)

5. Procédé selon l'une quelconque des revendications 1 à 4, **caractérisé en ce que** l'on utilise, en tant qu'agent de sulfoéthylation, le carbylsulfate.

6. Procédé selon l'une quelconque des revendications 1 à 4, **caractérisé en ce que** l'on utilise, en tant qu'agent de sulfoéthylation, l'acide éthionique ou son sel.

7. Procédé selon l'une quelconque des revendications 1 à 4, **caractérisé en ce que** l'on utilise, en tant qu'agent de sulfoéthylation, l'acide vinylsulfonique ou le vinylsulfonate.

8. Procédé selon la revendication 7, **caractérisé en ce que** l'on utilise le vinylsulfonate sous la forme d'une solution aqueuse de sel sodique de l'acide vinylsulfonique à une concentration de 10% à 40% en poids, pouvant être obtenue à l'échelle industrielle.

9. Procédé selon la revendication 8, **caractérisé en ce que** l'on ajuste et maintient le pH par addition d'acide.

10. Procédé selon la revendication 9, **caractérisé en ce que** l'on utilise, en tant qu'acide, l'acide sulfurique, lequel est ajouté en une seule partie.
